# EUROPEAN PATENT SPECIFICATION

(11) **EP 3 914 726 B1**
(45) Date of publication and mention of the grant of the patent: **06.11.2024**
(21) Application number: 20744302.9
(22) Date of filing: 23.01.2020
(51) Int. Cl.: A61K 38/17, A61P 35/00

(54) **MEDICAL USE OF VGAF**
MEDIZINISCHE VERWENDUNG VON VGAF
UTILISATION MÉDICALE DU VGAF

(30) Priority: 24.01.2019 IN 201911002903
(43) Date of publication of application: 01.12.2021
(73) Proprietor: Council of Scientific & Industrial Research, New Delhi 110001 (IN)
(72) Inventor: SRIVASTAVA, Avinash, Hyderabad, Telangana 500007 (IN); MISHRA, Rakesh K., Hyderabad, Telangana 500007 (IN)
(74) Representative: Graf von Stosch Patentanwaltsgesellschaft mbH
(86) International application number: PCT/IN2020/050073
(87) International publication number: WO 2020/152712

(56) References cited:
- WO-A2-03/001985
- WO-A2-03/074007
- EA-B1- 019 599
- US-A1- 2005 084 936
- US-A1- 2008 113 360
- WIDOM R L ET AL: "Cloning and characterization of hcKrox, a transcriptional regulator of extracellular matrix gene expression", GENE, ELSEVIER AMSTERDAM, NL, vol. 198, no. 1-2, 1 October 1997 (1997-10-01), pages 407 - 420, XP004570121, ISSN: 0378-1119, DOI: 10.1016/S0378-1119(97)00360-0
- MATHARU N K ET AL: "Vertebrate Homologue of Drosophila GAGA Factor", JOURNAL OF MOLECULAR BIOLOGY, ACADEMIC PRESS, UNITED KINGDOM, vol. 400, no. 3, 16 July 2010 (2010-07-16), pages 434 - 447, XP027125524, ISSN: 0022-2836, [retrieved on 20100521], DOI: 10.1016/J.JMB.2010.05.010
- DATABASE Genbak [online] NCBI; 9 January 2008 (2008-01-09), WAKAMATSU A ET AL: "unnamed protein product [Homo sapiens] - Protein - NCBI", XP055950871, retrieved from https://www.ncbi.nlm.nih.gov/protein/baf84308.1 Database accession no. BAF843008
- MARIANI FRANCESCO ET AL: "Th Inducing POZ-Kruppel Factor (ThPOK) Is a Key Regulator of the Immune Response since the Early Steps of Colorectal Carcinogenesis", PLOS ONE, vol. 8, no. 1, 17 January 2013 (2013-01-17), pages e54488, XP055950880, DOI: 10.1371/journal.pone.0054488
- PETER ARENBERGER, ARENBERGEROVA MONIKA, VOHRADNIKOVA OLGA, KREMEN JAROMIR: "Early Detection of Melanoma Progression by Quantitative Real-time RT-PCR Analysis for Multiple Melanoma Markers", KEIO JOURNAL OF MEDICINE, vol. 57, no. 1, March 2008 (2008-03-01), pages 57 - 64, XP055727926
- ZAENKER ET AL.: "A diagnostic autoantibody signature for primary cutaneous melanoma", ONCOTARGET, vol. 9, no. 55, 2018, pages 30539 - 30551, XP055724800, DOI: 10.18632/oncotarget.25669
- YINXIAN WEN; JING LI; LINLONG WANG; KAI TIE; JACQUES MAGDALOU; LIAOBIN CHEN; HUI WANG: "UDP-glucose dehydrogenase modulates proteoglycan synthesis in articular chondrocytes: its possible involvement and regulation in osteoarthritis", ARTHRITIS RESEARCH & THERAPY, vol. 16, no. 6, 3 December 2014 (2014-12-03), pages 484, XP021210499

## Description

### FIELD OF INVENTION

The current invention relates to an isolated protein vGAF/ThPOK, and its role as a novel DNA repair protein and as an early stage biomarker for melanoma, more particularly for skin cutaneous melanoma. In particular, the present invention relates to the isolated polypeptide or the polynucleotide encoding the same and a pharmaceutical composition comprising said polypeptide for use in a therapeutical method of enhancing the repair of damaged DNA bases in a cell; or the polypeptide or polynucleotide encoding the same for use in a method of preventing melanoma or skin cutaneous melanoma and for use in a method of treating early stage melanoma or early stage skin cutaneous melanoma.

### BACKGROUND OF THE INVENTION AND DESCRIPTION OF RELATED ART

Melanoma is the malignancy of pigment containing cells, also known as melanocytes, and is located mainly in the skin. It is the deadliest form of skin cancer. Melanoma often resembles moles which are generally black or dark brown. Melanomas arise when skin cells multiply rapidly as a consequence of mutations in their DNA, a primary cause of which may be UV exposure. The major type of melanoma is cutaneous or skin cutaneous melanoma (SCM), which is also not a single neoplastic disease; it consists of several subtypes.

Although skin cutaneous melanoma represents a small proportion of all skin cancers, it is the cause of majority of skin cancer-related deaths. SCM is an unpredictable disease and the major cause of the disease is the UV induced DNA damage in the skin (Armstrong and Cust, (2017) Cancer Epidemiol. Jun; 48:147-156). The excessive metastatic propensity of melanoma makes it the deadliest form of skin cancer, yet the underlying mechanism of metastasis remains elusive. In the United States itself, the five-year survival rates is 98% among those with localized disease and 17% among those in whom spread has occurred. Moreover, melanoma cases increased by 194% from 1975 to 2011 in USA itself (Weinstein et al., (2014) J Clin Aesthet Dermatol. 2014 Jun; 7(6)). The histological diagnosis of SCM is difficult, and diagnosing early stage melanoma is currently a challenge, but is critical both for treatment and prognosis. Most of the biomarkers that are currently used to assist in the diagnosis of melanoma are specific only for melanocytic neoplasms, and it is difficult to diagnose early stage neoplasms that may become metastatic later. Given the poor prognosis for late stage melanoma, early stage biomarkers are needed to aid in both the diagnosis and prognosis of melanoma and to determine which patients merit more aggressive therapy (US patent No. US7615349, Wei, D et al. (2018) Mol Med Rep. Feb; 17(2): 2907-2914.)

WO 03/074007 A2 discloses compositions and methods for modulating leukocyte activation and nucleic acids and proteins which are capable of modulating leukocyte activation. A method for screening for a bioactive agent capable of modulating lymphocyte activation comprises: contacting a candidate bioactive agent to a lymphocyte comprising a recombinant nucleic acid encoding a protein selected from the group consisting of HcKrox (vGAF) protein, SPRY2 protein, NFAT3 protein, TRIO protein, GRAP protein, and PEA15 protein.

The present invention relates to the vGAF/ThPOK/c-Krox protein, which is encoded by the zbtb7b gene in mice and humans, and its previously unknown role as a DNA-repair protein that can be used for preventing and treating melanoma.

### SUMMARY OF THE INVENTION

The present invention is defined in the appended claims.

### BRIEF DESCRIPTION OF THE ACCOMPANYING DRAWINGS

Figure 1 depicts that vGAF is essential for efficient DNA repair and cell survival after UV induced DNA damage
Figure 2 represents an analysis of vGAF expression in skin cutaneous melanoma (SCM) samples from human subjects
Figure 3 depicts the analysis for expression data of DNA damage associated proteins in Skin cutaneous melanoma (SCM) vs. Normal skin tissues (NSM).
Figure 4 represents the results of western blots demonstrating efficient DNA repair after UV induced DNA repair with overexpression of vGAF.

### DETAILED DESCRIPTION OF THE INVENTION

Melanoma is the malignancy of pigment containing cells, also known as melanocytes, and is located mainly in the skin. The major type of melanoma is cutaneous or skin cutaneous melanoma (SCM), which consists of several subtypes.

The stage at presentation of melanoma has a significant impact on the course and prognosis of the disease. The staging of melanoma holds prognostic value and dictates the recommendation in treatment. Staging is determined by the tumor, node, and metastasis (TMN) system developed by the American Joint Committee on Cancer (AJCC) (Dickson, P V and Gershenwald, J E; (2011) Surg Oncol Clinics of North America January; 20(1): 1-17).

The histological diagnosis of SCM is difficult, and distinguishing benign nevi from those that represent early stage true melanoma is currently a challenge, but is critical both for treatment and prognosis. Most of the biomarkers that are currently used to assist in the diagnosis of melanoma are unable to distinguish between early stage melanoma and healthy tissue, thus delaying the clinical management of the malignancy.

The current invention relates to the vGAF/ThPOK/c-Krox protein, which is encoded by the zbtb7b gene in mice and humans, for use in a therapeutical method of enhancing the repair of damaged DNA bases in a cell, a method of preventing melanoma or skin cutaneous melanoma (SCM) occurrence in human skin cells, a method of treating early stage melanoma or early stage skin cutaneous melanoma (SCM) in a patient by its previously unknown role as a DNA-repair protein. The protein is also used in a pharmaceutical composition for preventing and treating melanoma.

### Definitions:

SCM- As used herein, the terms "skin cutaneous melanoma" and "cutaneous melanoma" and "SCM" are used interchangeably herein.

Melanoma status- The terms "melanoma status" and "melanoma stage" are used interchangeably herein, and refer to the stage/ status of the disease in the subject. Examples of types of melanoma stages include, but are not limited to, the subject's risk of melanoma, the presence or absence of disease, the stage of disease in a subject (e.g., stages 0 - IV and recurrent melanoma), and the effectiveness of treatment of disease. Other stages and degrees of each status are known in the art (Trotter SC, et al, Global Review of Melanoma Follow-up Guidelines. J Clin Aesthet Dermatol. 2013; 6(9):18-26.

As used herein, the terms "vGAF", "ThPOK", "T helper POK", "c-krox", "zbtb7b protein", and "vertebrate GAGA factor" are used interchangeably herein, and refer to an amino-acid sequence with at least 95% sequence identity to SEQ ID No.:3.

The vertebrate homologue of GAF (GAGA Factor) is the ThPOK/c-Krox/vGAF protein, which is encoded by the zbtb7bgene in mice and humans.

The zbtb7bgene encodes the GAF protein in vertebrates. The vGAF protein has an N-terminal BTB/POZ domain and four zinc finger domains at its C-terminus. The BTB/POZ domain is a protein-protein interaction motif found in a large number of proteins across eukaryotes. GAF has multifaceted roles in gene regulation.

ZBTB7B/vGAF/ThPOK has been shown to be essential for the proper development of the hematopoietic and adipose tissues. A recent study also points out the role of vGAF in the onset of lactation and in the production of milk lipids. vGAF (ZBTB7B/ThPOK) was first identified as a tissue-specific activator of collagen genes in mouse dermis (Galera et al Proc (1994) Natl Acad Sci USA. Sep 27; 91(20): 9372-9376, Li et al (2013) J Biol Chem. May 31; 288(22): 15537-15546.. (Srivastava, et al., Cell. Mol. Life Sci. (2018) 75:623-633). The remarkable functional versatility of vGAF, at least in part, may stem from the structural domains of the protein. The zinc-finger domains present at C-terminal of vGAF allow sequence-specific tethering of vGAF molecules at numerous loci in the genome, while, the N-terminal BTB/POZ domain being a protein-protein interaction domain endows GAF with an ability to recruit protein-complexes of diverse functionalities at these loci (Srivastava, et al. Cell. Mol. Life Sci. (2018) 75:623-633).

DNA damage caused by ultraviolet (UV) light can lead to mutations, carcinogenesis, and cell death (Ananthaswamy and Pierceall, Photochem.Photobiol., 52, 1119-1136 (1990), Ziegler et al., Photochem. Photobiol., 63, 432-435 (1996)). UV-induced DNA damage occurs frequently in DNA as the bases of nucleic acids absorb light in a range coincident with that of natural sunlight, making the bases susceptible to photochemically induced alterations (Patrick, In: Photochemistry and Photobiology of Nucleic Acids, Wang (ed.) Vol. II, Academic Press, New York, pp. 1-32 (1976)).Ultraviolet (UV) light is the principle known cause of basal and squamous cell carcinomas and possibly melanomas. UV light can also lead to mutations and cell death (Ananthaswamy and Pierceall, Photochem. Photobiol., 52, 1119-1136 (1990), Ziegler et al., Photochem. Photobiol., 63, 432-435 (1996)).

As used herein, "damaged base" and "damaged bases" refers to structural deviations in nucleoside-5'-monophosphates present in a eukaryotic cell's genomic DNA. Examples of types of DNA damages are given below, but are not limited to the examples given here. One type of structural deviation is a covalent joining of the adjacent pyrimidines through the formation of a cyclobutane ring structure at the C5 and C6 positions. Another type of structural deviation is an imidazole ring fragmentation of a purine (either adenine or guanine). The location of such structural deviations in a cell's genomic DNA is referred to as a "lesion." Damaged bases preferably arise from, for instance, UV radiation, ionizing radiation, oxidative stress, alkylation damage, and deamination. Examples of lesions include cis-syn and trans-syn II cyclobutane pyrimidine dimers, FapyA and FapyG (Lloyd, DNA Repair, 408, 159-170 ((1998), and Lloyd, Progress in Nucleic Acid Research and Molecular Biology, 62, 155-175 (1999)).

Cells possess several DNA repair systems to counteract the damage produced by endogenous and exogenous agents. Repair of DNA damage is significant in preventing genetic modifications involved in the progression of carcinogenesis. Inefficient repair systems facilitate an increased risk of developing cancer, supporting the prospective study of DNA repair in relation to the onset of cancer.

As used herein, the term "DNA repair protein" means a protein that can enhance the repair of DNA base mutagenic damage. As used herein the term encompasses proteins that can directly act as DNA repair enzymes and also encompasses proteins that may increase the DNA repair activity in the cell by any other mechanism. The "DNA repair protein" may increase the DNA repair in the cell(s) by any mechanism, examples of which include, but are not limited to, recruiting DNA repair enzymes to the damaged DNA, increasing the activity of the DNA repair enzyme, or functioning as a DNA repair enzyme by binding to DNA itself.

"DNA repair enzymes" that repair DNA by binding to damaged DNA are often categorized by the type of DNA damage they repair, for example BER (base excision repair) enzymes, nucleotide excision repair (NER) enzymes; mismatch repair (MMR) enzymes; DNA helicases; DNA polymerases, and so on. For example, mutations such as 8-oxo-7,8-dihydro-2'-deoxyguanosine may be repaired by OGG1 (8-oxoGuanine glycosylase); T-T dimers which may be repaired by (Nucleotide excision repair (NER) Photolyase); 6-4 photoproducts (which may be repaired by NER); and 06-methyl guanine (which may be repaired by 06-alkyl guanine transferase (AGT)) (US20120219537A1). Many DNA repair enzymes have been disclosed in U.S. Pat. Nos. 5,077,211; 5,190,762; 5,272,079; and 5,296,231.

As used herein, "effective amount" means an amount of the compound, carrier, or of the composition sufficient to induce a desired effect, but low enough to avoid serious side effects. The effective amount of vGAF would be amount that is enough to enhance DNA repair activity in the cells it is applied to, which is required for prevention or treatment of melanoma, in particular, of cutaneous melanoma.

A "biomarker" in the context of the present disclosure is a molecular indicator of a specific biological property, such as a specific disease or malignancy; a biochemical feature or facet that can be used to measure the progress of disease or the effects of treatment.

The phrase "differentially present" refers to differences in the quantity and/or the frequency of expression of any gene, specifically of a biomarker present in a sample taken from test subjects or patients, as compared to a control subject or a reference subject or sample. A biomarker can be a polypeptide or a nucleic acid, which is detected at a higher frequency or at a lower frequency in samples of human melanoma subjects compared to samples of control subjects.

A biomarker can be differentially present in terms of quantity, frequency or both.

A polypeptide is differentially present between two samples if the amount of the polypeptide or nucleic acid in one sample is statistically significantly different from the amount of the polypeptide or nucleic acid in the other sample. For example, a polypeptide or nucleic acid is differentially present between the two samples if it is present at least about 25%, at least about 50%, at least about 75%, at least about 100%, at least about 120%, at least about 130%, at least about 150%, at least about 180%, at least about 200%, at least about 300%, at least about 500%, at least about 700%, at least about 900%, or at least about 1000% greater than it is present in the other sample, or if it is detectable in one sample and not detectable in the other.

For example, a polypeptide or nucleic acid is differentially present between the two sets of samples if it is detected at least about 25%, at least about 50%, at least about 75%, at least about 100%, at least about 120%, at least about 130%, at least about 150%, at least about 180%, at least about 200%, at least about 300%, at least about 500%, at least about 700%, at least about 900%, or at least about 1000% more frequently or less frequently observed in one set of samples than the other set of samples.

"Diagnostic" means identifying the presence or nature of a pathologic condition, in the current description the pathologic condition refers to melanoma. Melanoma may be subcutaneous skin melanoma as the pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives". Subjects who are not diseased and who test negative in the assay, are termed "true negatives."

As used herein, the term "specificity" is the percentage of subjects correctly identified as having a particular disease i.e., normal or healthy subjects. For example, the specificity is calculated as the number of subjects with a particular disease as compared to non-melanoma subjects (e.g., normal healthy subjects). The specificity of the assays described herein may range from about 80% to 100%. Preferably the specificity is about 90%, 95%, or 100%.

While a particular diagnostic method may not provide a definitive diagnosis of a condition, it suffices if the method provides a positive indication that aids in diagnosis.

Differential expression of biomarker can be seen by either measuring the biomarker levels in absolute amount for example., ng/ml, µg/ml or any other such absolute measurement) or a relative amount (for example, relative intensity of signals between the test and control samples).

The terms "affected tissue" and "patient tissue" are used interchangeably herein and refer to tissue from a melanoma subject, or a suspected melanoma subject. It may be from a mole, from (e.g., skin (epidermis and/or dermis) lymph nodes, metastatic sites, e.g., brain, lung, bone, liver, skin) or melanocytes).

The terms "unaffected tissue" and "control sample" and "healthy sample" as used herein refer to a tissue from a healthy subject or from healthy tissue that is known not to be affected by melanoma. It may be from a healthy person, or, portion of tissue taken from a patient, that does not have gross disease present, for example tissue that is about 1, 2, 5, 10, 20 or more cm from diseased tissue.

A "sample" as defined herein is any biological material obtained from an individual. A sample from a patient refers to a sample from a person who needs confirmed diagnosis for presence or absence of melanoma, or may require confirming a melanoma stage.

### Subject/ Patient Types:

Samples are collected from subjects to establish melanoma status or stage. The subjects may be subjects who have been determined to have a high risk of melanoma based on their family history, a previous treatment, subjects with physical symptoms known to be associated with melanoma, subjects identified through screening assays (e.g., routine melanoma screening) or other techniques. Other subjects may include subjects who have melanoma and the test is being used to determine the effectiveness of therapy or treatment they are receiving. Also, subjects may include healthy people who are having a test as part of a routine examination, or to establish baseline levels of the biomarkers. Samples may be collected from subjects who had been diagnosed with melanoma and received treatment to eliminate the melanoma, or perhaps are in remission. As used herein, "melanoma biopsy" refers to tissue from suspected melanoma, tissue from the edges of suspected melanoma or from normal tissue.

Types of Samples and Preparation of the Sample: The level of biomarker can be measured in different types of biological samples. The sample is preferably a biological tissue or fluid sample. Examples of biological tissue sample include, but are not limited to, blood or biopsy sample, from for example a melanoma biopsy. Examples of a biological fluid sample useful in this invention include blood, blood serum, plasma, vaginal secretions, urine, tears, saliva, urine, tissue, cells, organs, seminal fluids, bone marrow, cerebrospinal fluid, etc.Skin tissue from patient or control subjects may be the samples used for differentiating early stages of melanoma.

Reference or control tissue or cells may be normal cells (cells that are not melanoma cells) from a healthy subject. The reference cells may be primary cultured cells, fresh blood cells, established cell lines or other cells determined to be appropriate to one of skill in the art. The vGAF biomarker may be used to differentiate between control samples and early stage melanoma. The vGAF biomarker may be used to differentiate between control samples and early stage SCM.

"Gene" refers to a polynucleotide sequence that comprises sequences that are expressed in a cell as RNA and control sequences necessary for the production of a transcript or precursor.

A gene expression product analyzed according to a method described herein can be encoded by a full-length coding sequence or by any portion of the coding sequence.

"Polynucleotide" refers to a polymeric form of nucleotides of any length, either ribonucleotides or deoxyribonucleotides. Thus, the term includes, but is not limited to, single-, double-, or multi-stranded DNA or RNA, genomic DNA, cDNA, DNA-RNA hybrids, or a polymer comprising purine and pyrimidine bases or other natural, chemically or bio chemically modified, non-natural, or derivatized nucleotide bases, as well as polynucleotides that have been modified in order to introduce a means for attachment (e.g., to a support).

The term vector may include e.g. plasmid vectors, viral vectors, cosmid vectors, or artificial chromosome vectors. Typically, a vector is capable of replication in a bacterial host, for instance *E*. *coli,* or in a eukaryotic cell. Preferably the vector is a plasmid.

Selection of a vector depends upon a variety of desired characteristics in the resulting construct, such as a selection marker, vector replication rate, and the like. Suitable host cells for cloning or expressing the vectors herein are prokaryotic or eukaryotic cells. Suitable prokaryotes include eubacteria, such as gram-negative or gram-positive organisms, for example, *E*. *coli.*

An expression vector optionally includes regulatory sequences operably linked to the coding region. Any particular promoter, any promoter known in art, and suitable promoter may be used. The promoter can be a constitutive or an inducible promoter. It may be heterologous with respect to the host cell.

An expression vector may include a ribosome binding site (a Shine Dalgarno site for prokaryotic systems or a Kozak site for eukaryotic systems) and a start site (e.g., the codon ATG) to initiate translation of the transcribed message to produce the enzyme. It can also include a termination sequence to end translation. The vector may encompass any of the termination sequences known in art.

The present disclosure also encompasses expression vectors that provide for transient expression in eukaryotic cells of a coding sequence encoding a polypeptide used according to the invention. Methods for the transient expression of coding regions are well known in the art.

The polynucleotide used to transform the host cell optionally includes one or more marker sequences, which typically encode a molecule that inactivates or otherwise detects or is detected by a compound in the growth medium. For example, the inclusion of a marker sequence can render the transformed cell resistant to an antibiotic, or it can confer compound-specific metabolism on the transformed cell. Examples of a marker sequence are sequences that confer resistance to kanamycin, ampicillin, chloramphenicol, tetracycline, and neomycin.

The polypeptides for use of the present invention may also include targeting sequence, preferably, an exogenous targeting sequence. As used herein, a "targeting sequence" is a polypeptide that is fused to a peptide or polypeptide for use of the current invention, if required.

vGAF polypeptide may be targeted to the nucleus without any exogenous targeting sequence. The targeting sequence may be a mitochondria localization sequence (MLS) that causes migration into mitochondria.

Whether a polypeptide for use of the present invention is delivered to the appropriate organelle can be determined by several methods. The polypeptide can be introduced to a eukaryotic cell by, for instance, microinjection of the polypeptide into the cytoplasm of the cell. Alternatively, the polypeptide may be introduced to the cytoplasm of the cell as a composition including the polypeptide and a pharmaceutically acceptable carrier such as a liposome, phospholipid, or pH-activated lipid. Pharmaceutically acceptable carriers are described herein. To determine whether the introduced polypeptide is targeted to the nucleus or the mitochondria of a cell, the appropriate organelle can be isolated, and the amount of the polypeptide in the organelle determined. Alternatively, immunofluorescence analysis with antibody that binds to the polypeptide can be used to determine the intracellular distribution of the polypeptide after it is introduced.

Whether the polypeptide of the present invention retains vGAF or DNA repair activity, once transported into the organelle can be determined by several methods. To measure activity after introduction to the cell, the appropriate organelle can be isolated, the polypeptide isolated from the organelle, and the activity of the isolated polypeptide determined. Alternatively, the repair rate of damaged DNA in the cell can be determined using, for instance, coding sequence-specific repair assays, photoproduct removal, and/or quantitative PCR.

SEQ ID No.: 1 relates to the vGAF polynucleotide sequence of homo sapiens.

Polynucleotides encoding a polypeptide of the present invention, for example, a polynucleotide encoding a vGAF polypeptide is used in the present invention. The vGAF protein disclosed herein can be used as biomarker (not claimed) and a DNA repair protein for diagnostic (not claimed) or treatment methods disclosed herein.

The parent sequence of vGAF is SEQ ID No.: 1. In one embodiment, the polynucleotides of the present invention have at least 95% sequence identity to SEQ ID No.: 2.The polynucleotide used in the current invention encodes for a polypeptide with at least 95% identity to SEQ ID No.: 3. A polynucleotide may include nucleotide sequences having different functions, including for instance coding sequences, and non-coding sequences such as regulatory sequences. A regulatory sequence is a nucleotide sequence that regulates expression of a coding region to which it is operably linked. Examples of regulatory sequences may include e.g. promoters, transcription initiation sites, translation start sites, translation stop sites, and terminators.

"Operably linked" refers to a juxtaposition wherein the components so described are in a relationship permitting them to function in their intended manner. A regulatory sequence is "operably linked" to a coding region when it is joined in such a way that expression of the coding region is achieved under conditions compatible with the regulatory sequence.

The vGAF protein, which has an amino acid sequence that has at least 95% identity with SEQ ID No.: 3 is a DNA repair protein.

The vGAF protein disclosed herein can be used in combination with another one or more than one DNA repair protein for diagnostic (not claimed) or treatment methods disclosed herein.

Methods and compositions are disclosed (not claimed) herein for evaluating tissue samples from patients (for example, cutaneous tissue samples, which may be samples from suspected compound nevi/moles, primary melanomas) to diagnose melanoma, specifically early stage SCM in patients. The ability to diagnose samples with a high degree of accuracy using vGAF as the biomarker and sensitivity facilitates determination of melanoma progression at an early stage. Stage 0 or stage 1 melanoma can be diagnosed by using vGAF as a predictive biomarker.

Stage 0 melanoma can be distinguished from healthy skin by using vGAF as the predictive biomarker.

Diagnosing early stage melanoma, at stage 0 or stage 1, by distinguishing from healthy tissue is highly critical since subsequent clinical decisions such as whether or not to undergo further surgery or other treatment modalities, is affected by early diagnosis, and before the melanoma has had a chance to metastasize.

Any technique can be used for analyzing vGAF gene expression, including those that measure RNA or protein expression. A sample from a skin sample from a patient can be collected and processed to obtain RNA, protein, or tissue sections, to produce a test sample for analysis. The relative expression of vGAF RNA or protein is determined by any of the methods available, and is compared to vGAF RNA or protein from a control sample, which is a skin tissue sample from a healthy person. The same assay is used to determine vGAF expression in both the patient and healthy samples. The values obtained for the test sample characterize the sample as malignant or not.

The test samples may be, for example, skin tissues from patients who have been exposed to high dosages of UV light, surgically collected tumors, compound nevi, collected in a manner that preserves RNA, or alternatively fixed (e.g., formalin fixed) and embedded in paraffin prior to analysis, preserved by flash freezing or fixation, and/or treated with an RNA Stabilization Reagent.

Expression of a control gene may be monitored as part of the assay. Examples of genes whose expression can be monitored as control to ensure equal sample volumes used, equal reaction efficiency etc., and to ensure minimization of errors may include e.g. histones and collagen genes.

Commonly used methods are for the quantification of mRNA expression in a sample include northern blotting and *in situ* hybridization (Parker & Barnes, Methods in Molecular Biology 106:247-283, 1999); RNAse protection assays (Hod, Biotechniques 13:852 854, 1992); and PCR-based methods, such as reverse transcription polymerase chain reaction (RT-PCR) (Weis et al., Trends in Genetics 8:263 264, 1992). Alternatively, antibodies may be employed that can recognize specific duplexes, including DNA duplexes, RNA duplexes, and DNA-RNA hybrid duplexes or DNA-protein duplexes.

### PCR-Based Methods (not claimed)

vGAF expression in samples from patients and from healthy tissues as controls, to diagnose melanoma, specifically SCM, can be analyzed by PCR (not claimed).

The diagnosis can be done to diagnose early stage SCM in patients, by determining vGAF expression. PCR is useful to amplify and detect transcripts from a melanoma sample.

Any of the PCR methodologies well known in art, can be used for vGAF gene expression analyses by RNA quantitation.

Any sets of vGAF primers can be used for determining vGAF expression through RNA expression analyses such as Reverse Transcriptase PCR (RT-PCR).

The vGAF forward and reverse primers with SEQ ID No.:4 and SEQ ID No.:5 may be used.

Reverse Transcriptase PCR (RT-PCR) is a sensitive quantitative method that can be used to compare vGAF mRNA levels in different samples (such as skin samples from patients and from healthy controls, such as benign nevi and melanoma samples.

To perform RT-PCR, mRNA is isolated from a sample (e.g., total RNA isolated from a human patient mRNA is extracted, from the patient and control samples. The samples can be for example, from frozen or archived paraffin-embedded and fixed (e.g. formalin-fixed) tissue samples. Methods for mRNA extraction are well known in the art, such as given in Ausubel et al., Current Protocols in Molecular Biology, John Wiley and Sons, 1997; methods for RNA extraction from paraffin embedded tissues are disclosed, for example, in Rupp and Locker, Lab Invest. 56: A67, 1987, and De Andres et al., Biotechniques 18:42044, 1995. Next, RNA is reverse transcribed into cDNA. The cDNA is amplified in a PCR reaction. Any of the reverse transcriptase's known in art can be used. Examples of reverse transcriptase's may include e.g. avian myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the conditions and desired readout. The derived cDNA can then be used as a template in the subsequent PCR reaction. Guidelines for PCR primer and probe design are described in Dieffenbach et al., "General Concepts for PCR Primer Design' in: PCR Primer, A Laboratory Manual, Cold Spring Harbor Laboratory Press, New York, 133-155, 1995: Innis and Gelfand, "Optimization of PCRs' in: PCR Protocols, A Guide to Methods and Applications, CRC Press, London, 5-11, 1994; and Plasterer, T. N. Primer select: Primer and probe design. Methods Mol. Biol. 70:520-527, 1997. Factors considered in PCR primer design include primer length, melting temperature (Tm), and G/C content, specificity, complementary primer sequences, and 3'-end sequence. For quantitative PCR, a third oligonucleotide, or probe, can be used to detect nucleotide sequence located between the PCR primers.

A variation of the RT-PCR technique is real time quantitative PCR, which measures PCR product accumulation through a dual-labeled fluorigenic probe (i.e., TaqManTM probe). Real time PCR is compatible both with quantitative competitive PCR, where internal competitor for each target sequence is used for normalization, and with quantitative PCR using a normalization gene contained within the sample, or a housekeeping gene for RT-PCR. For further details see, e.g. Held et al., Genome Res. 6:986-994, 1996. 35. Gene expression can be examined using fixed, paraffin embedded tissues as the RNA source.

Methods of examining expression in fixed, paraffin-embedded tissues, are described in Godfrey et al., J.; Molec. Diagn.45 2: 84-91, 2000; and Spechtet.al., Am. J. Pathol. 158: 419-29, 2001.

### Protein Detection Methodologies (not claimed):

Immunohistochemical methods can also be used for detecting vGAF expression in the patient samples. Antibodies, most preferably monoclonal antibodies, specific for a gene product may be used to detect expression. The antibodies can be detected by direct labelling of the antibodies themselves, for example, with radioactive labels, fluorescent labels, hapten labels such as, biotin, or an enzyme such as horse radish peroxidase or alkaline phosphatase. Also, unlabelled primary antibody may be used in conjunction with a labelled secondary antibody, comprising antisera, polyclonal antisera or a monoclonal antibody specific for the primary antibody. Immunohistochemistry protocols and kits are well known in the art and are commercially available.

Disclosed herein is a diagnostic kit for early detection of melanoma (not covered by the claims). The kit comprising of:
a) a pool of forward and reverse primer sets having:

| | |
|---|---|
| Set 1 being | Forward primer having SEQ ID No.4 |
| | Reverse primer having SEQ ID No.5; |
| Set 2 being | Forward primer having SEQ ID No.6 |
| | Reverse primer having SEQ ID No.7; |
| Set 3 being | Forward primer having SEQ ID No.8 |
| | Reverse primer having SEQ ID No.9; |

wherein the said primers are used alone or in combination,
b) reagents for carrying out real time PCR;
c) reverse transcriptase enzyme;
d) an instruction manual for using the kit.

The isolated polypeptide comprising an amino acid sequence with at least 95% identity to SEQ ID No.:3, wherein the polypeptide has DNA repair activity is used in the present invention.

In one embodiment the isolated polypeptide with DNA repair activity encoded by a nucleotide sequence with at least 95% identity to SEQ ID No.: 2. The DNA repair protein disclosed herein is vGAF/thPOK protein.

The isolated polypeptide amino acid sequence with at least 95% identity to SEQ ID No.:3, is downregulated in melanoma patients, compared to healthy controls.

The isolated polypeptide amino acid sequence with at least 95% identity to SEQ ID No.:3 is downregulated in skin cutaneous melanoma (SCM) patients, compared to healthy controls.

The isolated polypeptide disclosed herein may be used as a biomarker for detecting early stage melanoma (not claimed). It may also be used as a biomarker for detecting melanoma at any stage of progression (not claimed).

The isolated polypeptide disclosed herein may be used as a biomarker for detecting early stage SCM (not claimed). It may also be used as a biomarker for detecting SCM at any stage of progression (not claimed).

It may also be used as a single biomarker for detecting early stage melanoma, without the need for any other biomarker (not claimed).

It may also be used as a single biomarker for detecting early stage SCM, without the need for any other biomarker (not claimed).

It may also be used as a single biomarker for detecting SCM at any stage of progression (not claimed).

A method of detecting early stage melanoma in a patient is disclosed herein (not claimed), the method comprising the step of determining expression level of vGAF in test samples comprising skin tissue from the patient and comparing it to the vGAF expression levels to samples comprising healthy skin tissue, wherein a decrease in the level of vGAF expression in the test samples from the patient indicates occurrence of melanoma in the patient.

The step of determining expression level of vGAF may be carried out by determining the vGAF mRNA expression levels.

A method of detecting early stage skin cutaneous melanoma in a patient is described herein (not claimed), the method may comprise the step of determining expression level of vGAF mRNA in test samples comprising skin tissue from the patient and comparing it to the vGAF mRNA expression levels in samples comprising healthy skin tissue, wherein a decrease in the level of vGAF mRNA expression in the test samples from the patient indicates occurrence of skin cutaneous melanoma in the patient.

The method of detecting early stage SCM in a patient (not claimed) may further comprise the steps of:
(a) isolating mRNA from the test samples comprising skin tissue from the patient and the samples comprising healthy skin tissue,
(b) detecting vGAF mRNA expression level in both the sets of isolated mRNA by doing RT-PCR using a primer set comprising a forward primer and a reverse primer;
(c) comparing the expression level of the test sample with the normal skin tissue,
wherein lower level of mRNA in the test sample indicates SCM.

The method described above, wherein the primer set in step (b) may comprise a forward primer with SEQ ID No.: 4 and a reverse primer with SEQ ID No: 5.

The step of determining expression level of vGAF may be done by determining the vGAF protein expression levels.

A method of detecting early stage melanoma in a patient is described herein (not claimed), the method may comprise the step of determining expression level of vGAF protein in at least one test sample comprising skin tissue from the patient and comparing it to the vGAF protein expression levels to at least one sample comprising healthy skin tissue, wherein a decrease in the level of vGAF protein expression in at least one test sample from the patient indicates occurrence of melanoma in the patient.

A method of detecting early stage SCM in a patient is also disclosed herein (not claimed), the method may comprise the step of determining expression level of vGAF protein in at least one test sample comprising skin tissue from the patient and comparing it to the vGAF protein expression levels to at least one sample comprising healthy skin tissue, wherein a decrease in the level of vGAF protein expression in the at least one test sample from the patient indicates occurrence of SCM in the patient.

The method of detecting early stage melanoma in a patient (not claimed) may further comprise the steps of:
(a) treating the at least one test sample comprising skin tissue from the patient and the at least one sample comprising healthy skin tissue with at least one antibody against vGAF protein;
(b) detecting vGAF protein expression level in the at least one test sample comprising skin tissue from the patient and the at least one sample comprising healthy skin tissue from step (a) by detecting amount of the at least one antibody bound to the vGAF protein molecules in the samples; and
(c) comparing the expression level of the vGAF protein in the at least one test sample and the at least one sample from the healthy skin tissue;
wherein lower level of vGAF protein in the at least one test sample compared to the at least one sample from healthy skin tissue indicates melanoma.

The method of detecting early stage SCM in a patient (not claimed) may further comprise the steps of:
(a) treating at least one test sample comprising skin tissue from the patient and at least one sample comprising healthy skin tissue with at least one antibody against vGAF protein;
(b) detecting vGAF protein expression level in the at least one test sample comprising skin tissue from the patient and the at least one sample comprising healthy skin tissue from step (a) by detecting amount of the at least one antibody bound to the vGAF protein molecules in the samples; and
(c) comparing the expression level of the vGAF protein in at least one test sample and at least one sample from the healthy skin tissue; wherein lower level of vGAF protein in at least one test sample compared to at least one sample from healthy skin tissue indicates occurrence of SCM.

The antibody detection in step (b) can be done by any of the known methods for immunodetection known in art, examples of which may include e.g. ELISA, immunofluorescence or western blotting.

A diagnostic kit for detecting early stage melanoma is disclosed herein (not claimed), comprising reagents for doing real time PCR detection of vGAF mRNA in test samples comprising skin tissue from patients and samples comprising healthy skin tissue, wherein the kit comprises a first forward and a first reverse primer and reverse transcriptase enzyme.

A diagnostic kit for detecting early stage skin cutaneous melanoma is also described herein (not claimed), comprising reagents for doing real time PCR detection of vGAF mRNA in test samples comprising skin tissue from patients and samples comprising healthy skin tissue, wherein the kit comprises a first forward and a first reverse primer and reverse transcriptase enzyme.

The first forward primer may comprise SEQ ID No.:4 and the first reverse primer may comprise SEQ ID No.:5 in the diagnostic kit.

The diagnostic kit may further comprise a second forward primer and a second reverse primer for amplifying the mRNA of a control mRNA.

The second forward primer may comprise SEQ ID No.: 6 and the second reverse primer may comprise SEQ ID No.: 7, and wherein the control mRNA may be histone H2.

The second forward primer may comprise SEQ ID No.: 8 and the second reverse primer may comprise SEQ ID No.: 9, and wherein the control mRNA may be collagen.

According to the current invention, the isolated polypeptide comprising an amino acid sequence with at least 95% identity to SEQ ID No.:3 is used in a therapeutical method of enhancing the repair of damaged DNA bases in a cell, the method comprising the step of introducing an effective amount of an isolated vGAF polypeptide into the cell, wherein the effective amount of polypeptide is sufficient to enhance the repair of damaged DNA in the cell. In one embodiment, the cell has been exposed to, or is at risk of being exposed to a DNA damaging agent.

In one embodiment, the step of introducing the isolated vGAF polypeptide in the cell can be done by expressing a polynucleotide encoding vGAF polypeptide.

Another embodiment is the isolated polypeptide comprising an amino acid sequence with at least 95% identity to SEQ ID No.:3 for use in a method of preventing melanoma occurrence in human skin cells, the method comprising the step of introducing an effective amount of an isolated vGAF polypeptide into the human skin cells, wherein the effective amount of vGAF polypeptide is sufficient to enhance the repair of damaged DNA in the human skin cells, and wherein the human skin cells have been exposed to or are at risk of being exposed to a DNA damaging agent.

In another embodiment, the isolated polypeptide, which comprises an amino acid sequence with at least 95% identity to SEQ ID No.: 3 for use in a method of treating early stage melanoma in a patient is provided. The method comprising the step of introducing an effective amount of an isolated vGAF polypeptide into the early stage melanoma cells, wherein the effective amount of polypeptide is sufficient to enhance the repair of damaged DNA in the early stage melanoma cells, and wherein the isolated polypeptide comprises an amino acid sequence with at least 95% identity to SEQ ID No.:3.

In another embodiment, the isolated polypeptide, which comprises an amino acid sequence with at least 95% identity to SEQ ID No.: 3 for use in a method of preventing skin cutaneous melanoma (SCM) occurrence in human skin cells is provided. The method comprising the step of introducing an effective amount of an isolated vGAF polypeptide into the human skin cells, wherein the effective amount of polypeptide is sufficient to enhance the repair of damaged DNA in the human skin cells, and wherein the isolated polypeptide comprises an amino acid sequence with at least 95% identity to SEQ ID No.:3 and wherein the human skin cells have been exposed to or are at risk of being exposed to a DNA damaging agent.

In another embodiment, the isolated polypeptide, which comprises an amino acid sequence with at least 95% identity to SEQ ID No.: 3 for use in a method of treating early stage skin cutaneous melanoma (SCM) in a patient is provided. The method comprising the step of introducing an effective amount of an isolated vGAF polypeptide into the early stage melanoma cells, wherein the effective amount of polypeptide is sufficient to enhance the repair of damaged DNA in the early stage melanoma cells, and wherein the isolated polypeptide comprises an amino acid sequence with at least 95% identity to SEQ ID No.:3.

In one embodiment, the present invention encompasses the isolated polypeptide, which comprises an amino acid sequence with at least 95% identity to SEQ ID No.: 3 for use in a method of preventing skin cutaneous melanoma (SCM) occurrence in human skin cells, the method comprising the step of introducing an effective amount of an isolated polypeptide into the human skin cells, wherein the effective amount of polypeptide is sufficient to enhance the repair of damaged DNA in the human skin cells, and wherein the isolated polypeptide comprises an amino acid sequence with at least 95% identity to SEQ ID No.:3 and wherein the human skin cells have been exposed to or are at risk of being exposed to a DNA damaging agent.

A diagnostic kit for early detection of melanoma is described herein (not claimed), the kit may comprise:
a) a pool of forward and reverse primer sets having:

| | |
|---|---|
| Set 1 being | Forward primer having SEQ ID No.4 |
| | Reverse primer having SEQ ID No.5; |
| Set 2 being | Forward primer having SEQ ID No.6 |
| | Reverse primer having SEQ ID No.7; |
| Set 3 being | Forward primer having SEQ ID No.8 |
| | Reverse primer having SEQ ID No.9; |

wherein the said primers are used alone or in combination,
b) reagents for carrying out real time PCR;
c) reverse transcriptase enzyme;
d) an instruction manual for using the kit.

In another embodiment, the invention is directed to the isolated polypeptide, which comprises an amino acid sequence with at least 95% identity to SEQ ID No.: 3 for use in a therapeutical method for enhancing the repair of damaged bases in a cell, preferably a skin cell. The method comprises: introducing to a skin cell exposed to or at risk of exposure to an agent that damages DNA a composition that includes an effective amount of a composition including a vGAF polypeptide, or a polynucleotide encoding a vGAF polypeptide, where it may include a targeting sequence. Increased level of mutagenesis may lead to the increased presence of damaged DNA, increased mutagenesis rates, increased immunosuppression, increased tumor formation (for instance, increased actinic keratosis, increased basal cell carcinoma, and increased squamous cell carcinoma, and possibly increased melanoma), and increased incidence of apoptotic cells.

In one embodiment, the present invention provides a pharmaceutical composition that contains the vGAF polypeptide and a pharmaceutically acceptable carrier for use in a method of prevention or treatment of melanoma as defined in claim 5.

The compositions for use of the present invention include a pharmaceutically acceptable carrier. Typically, the composition includes a pharmaceutically acceptable carrier when the composition is used as for prevention or treatment of melanoma, specifically SCM. The compositions for use of the present invention may be formulated in pharmaceutical preparations in a variety of forms adapted to the chosen route of administration. Formulations include those suitable for topical administration, parental administration (for instance intramuscular, intraperitoneal, or intravenous), oral, transdermal, nasal, or aerosol, preferably, topical.

The formulations used in the present invention may be conveniently packed as unit dosage form and may be prepared by methods well known in the art of pharmacy. Methods of preparing a pharmaceutical composition include the step of bringing the active compound (for example, vGAF polypeptide) into association with a carrier that constitutes one or more accessory ingredients. In general, the formulations are prepared by uniformly and intimately bringing the active compound into association with a liquid carrier, a finely divided solid carrier, or both, and then, if necessary, shaping the product into the desired formulations. The amount of active ingredient that is combined with the carrier materials to produce a single dosage form may depend upon the subject treated and the particular mode of administration.

The pharmaceutical composition used in the invention may be conveniently administered to the patient, at least once a day. A typical preparation may contain from about 5% to about 95% active compound (w/w). Preferably, such preparations contain from about 20% to about 80% active compound. The amount of active compound in such therapeutically useful pharmaceutical compositions is such that the dosage level will be effective to prevent or treat melanoma or SCM in human subjects.

The formulation used in this invention may also comprise a compound or compounds that deliver the active compound to the interior of cells, preferably to the interior of living skin cells under the skin's stratum corneum. Accordingly, such compounds deliver the active compounds across the stratum corneum and then across the outer cellular membrane of living cells. Examples of such compounds include liposomes, phospholipids, and pH-activated lipids (see, for example, U.S. Pat. No. 5,190,762).

Compositions or formulations that are suitable for topical administration of the vGAF polypeptide for prevention or treatment of melanoma or SCM, may include dusting powders, ointments, creams, gels or sprays for the administration of the active compound to cells, preferably skin cells. Such formulations may also comprise one or more than one of the following components, inorganic pigments, organic pigments, inorganic powder, organic powder, hydrocarbon, silicone, ester, triglyceride, lanolin, wax, animal or vegetable oil, surfactant, polyhydric alcohol, sugar, vitamin, amino acid, antioxidant, free radical scavenger, ultraviolet light blocker, sunscreen agents, preservative, fragrance, thickener, or combinations thereof.

The formulation may also encompass the application by topical administration, and the active compounds of the present invention can be used in cosmetic formulations (e.g., skincare cream, sunscreen, make-up products, and other dermatological compositions) in various pharmaceutical dosage forms, and in the form of oil-in-water or water-in-oil emulsions, solutions, gels, or vesicular dispersions. Examples of cosmetic formulations described herein include creams that can be applied either to the face or to the scalp and hair, as well as to the human body, in particular those portions of the body that are chronically exposed to sun. They may also serve as the base for a lipstick.

Cosmetic formulations usually include a lipid phase and often an aqueous phase too. Examples of lipid phase are mineral oils, mineral waxes oils, such as triglycerides of capric or of caprylic acid, castor oil; fats, waxes and other natural and synthetic fatty substances, esters of fatty acids with alcohols such as isopropanol, propylene glycol or glycerol, or esters of fatty alcohols with alkanoic acids of low carbon number or with fatty acids; alkyl benzoates; silicone oils, for example, dimethylpolysiloxanes, diethylpolysiloxanes, diphenylpolysiloxanes and mixed compositions made from these components described herein.

The step of introducing an effective amount of an isolated polypeptide into the human skin cells can be performed by incorporating the vGAF protein or functional peptides derived from the vGAF protein in a sunscreen.

The vGAF protein with at least 95% sequence identity to SEQ ID No.:3 may be part of a sunscreen. The composition may then contain other ingredients that will provide a cosmetically or pharmaceutically acceptable product.

The cosmetic formulation may be a sunscreen composition. A sunscreen can advantageously additionally include at least one further UVA filter and/or at least one further UVB filter and/or at least one inorganic pigment, for example an inorganic micropigment. The UVB filters can be oil-soluble or water-soluble. Advantageous oil-soluble UVB filter substances may be, for example: 3-benzylidenecamphor derivatives, such as 3-(4-methylbenzylidene)camphor and 3-benzylidenecamphor; 4-aminobenzoic acid derivatives, preferably 2-ethylhexyl 4-(dimethylamino)benzoate and amyl 4-(dimethylamino)benzoate; esters of cinnamic acid, such as 2-ethylhexyl 4-methoxycinnamate and isopentyl 4-methoxycinnamate; derivatives of benzophenone, such as 2-hydroxy-4-methoxybenzophenone, 2-hydroxy-4-methoxy-4'-methylbenzophenone and 2,2'-dihydroxy-4-methoxybenzophenone; esters of benzalmalonic acid, such as di(2-ethylhexyl) 4-methoxybenzalmalonate. Advantageous water-soluble UVB filter substances may be, for example: salts of 2-phenylbenzimidazole-5-sulphonic acid, such as its sodium, potassium or its triethanolammonium salt, and the sulphonic acid itself; sulphonic acid derivatives of benzophenones, such as 2-hydroxy-4-methoxybenzophenone-5-sulphonic acid and salts thereof; sulphonic acid derivatives of 3-benzylidenecamphor, such as, for example, 4-(2-oxo-3-bornylidenemethyl) benzenesulphonic acid, 2-methyl-5-(2-oxo-3-bornylidenemethyl) benzenesulphonic acid and salts thereof.

In another embodiment, the present invention is further directed to a polynucleotide for use in method of the invention that includes a coding sequence encoding a polypeptide having vGAF activity as defined by the claims. The vGAF coding polynucleotide may be operably linked to a targeting sequence. The vGAF coding polynucleotide may also be operably linked to a nuclear localization sequence. The polynucleotide comprises a nucleotide sequence having at least 95% identity with a nucleotide sequence of SEQ ID NO:2.

The present description provides a method for treating tumor formation in a subject (not claimed). The method comprises: introducing a composition that includes an effective amount of a polypeptide having vGAF activity.

In another embodiment, the present invention further provides the isolated polypeptide, which comprises an amino acid sequence with at least 95% identity to SEQ ID No.: 3 for use in methods for treating melanoma in a cell, by treating mutagenesis in a cell, preferably a skin cell, in response to an agent that damages DNA, preferably UV light. In this aspect of the invention, mutagenesis rates are decreased, because of increase in DNA repair activity in the cell by introduction of vGAF polypeptide, or functional vGAF peptides. The vGAF polypeptide used for treatment of mutagenesis has at least 95% sequence identity to SEQ ID No.: 3. Mutagenesis results when repair of damaged DNA does not occur and, upon replication of the DNA, a different base is inserted. The method includes introducing to a skin cell exposed to, or at risk of exposure to an agent that damages DNA, a composition that includes an effective amount of vGAF polypeptide, or vGAF CDS coding for vGAF polypeptide, that may include a targeting sequence in some embodiments. The rate of mutagenesis in a cell is reduced can be determined by assays known in art, for instance, HPRT mutagenesis assays (O'Neill et al, Mutat. Res., 45, 103-109 (1977)). The measurement of mutagenesis using an HPRT assay involves the selection of mammalian cells that are resistant to the killing effects of 6-thioguanine through a mutation in the HPRT coding sequence. The assay relies on an inability of HPRT-cells to activate 6-thioguanine for incorporation into DNA that results in cell death. All the cells with wild type HPRT are killed upon selection with 6-thioguanine. The rate of mutagenesis in cells treated with the vGAF polypeptide can be determined and compared to the rate of mutagenesis in untreated cells. The presence of a lower mutagenesis rate in treated cells relative to untreated cells indicates that the polypeptide enhances the DNA repair in the cell, and therefore decreases the mutagenesis rate of DNA. The cells can be *in vivo* or *ex vivo.*

Formulations comprising vGAF polypeptide or functional peptides derived from vGAF, also encompass compositions that are suitable for oral administration. Examples of compositions for oral administration may include e.g. tablets, capsules, lozenges, or cachets, solution or suspension in an aqueous liquor or non-aqueous liquid such as a syrup, an emulsion, each containing a predetermined amount of the active compound as a powder or granules, which may also include liposomes containing the active compound.

The above compositions for oral administration may comprise other components such as a binder, an excipient, a disintegrating agent, a lubricant and may also include a sweetening agent such as sucrose, fructose, lactose or aspartame; and a natural or artificial flavoring agent. The formulation may comprise other materials such as coatings, or bulking agents. For instance, tablets, pills, or capsules may be coated with gelatin, wax, shellac, or sugar and the like. The material used in preparing any unit dosage form is substantially nontoxic in the amounts employed. The vGAF polypeptide can also be incorporated into sustained-release preparations and devices.

In one embodiment, any composition comprising vGAF for used for the prevention or treatment of melanoma may comprise functional peptides derived from vGAF instead of the full length vGAF protein having at least 95% identity to SEQ ID NO: 3. The use of functional peptides instead of full-length polypeptide has been described many times in literature. US patent No. US20100093637 describes method of treating signs of aging on the skin, such as enhancing the elasticity of skin, enhancing the firmness of skin, smoothing the surface of the skin, and reducing the appearance of wrinkles on the skin, the method comprising applying to skin a composition comprising c-krox derived peptides, the peptides being derived from C-krox binding sequences of three zinc finger domains that are effective for stimulating the collagen synthesis of normal human dermal fibroblast, these peptides derived from c-krox can be functional and exert desired effects on skin by topical application.

### EXAMPLES

The following examples are given by way of illustration of the present invention

### Sources and materials used:

### Mouse resource:

ZBTB7B knockout heterozygous mice were purchased from Jacksons Laboratory USA (Stock No:027663). Mice were bred in Centre for Cellular & Molecular Biology (CCMB), Hyderabad, Animal house and progenies were genotyped for identifying the homozygous knockouts and wild type animals. Wild type littermates were used as controls whenever a homozygous mice was used in an experiment.

### Cells and tissue resource:

Wild type and ZBTB7B knockout mice were euthanized following the standard protocols approved by animal ethical committee. Small sections of skin were cut out and used as skin explants in all our experiments.

Lung tissue was isolated from the carcass of mice. Subsequently, fibroblast was isolated from these tissue pieces using standard cell culture techniques.

HEK cells used in the experiments were commercially procured earlier and maintained in the research laboratory.

RNA seq data for vGAF (ZBTB7B), XRCC1, XRCC5, XRCC6, XRCC7, APLF, APRATAXIN, PNKP, Lig4, Lig3, PARP2, and ATR.

RNA seq data is available in public domain on internet. The TCGA (The Cancer Genome Atlas) and GTEx (Genotype-Tissue Expression) databases were used for our analysis.

Expression-DIY module of GEPIA (Gene Expression Profiling Interactive Analysis) was used to analyze RNA-seq data for skin cutaneous melanoma and normal skin tissue.

### Example 1

### vGAF interacts with multiple DNA repair proteins and is essential for efficient DNA repair and cell survival after UV induced DNA damage

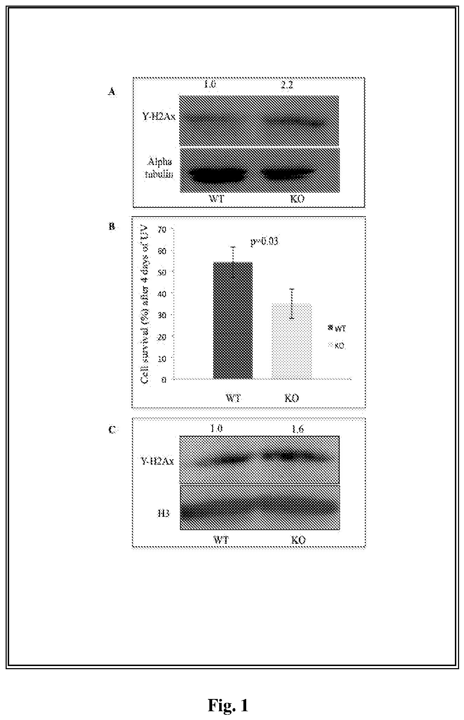

Cellular response to the DNA damage is mediated by DNA damage response (DDR) signaling pathways. ATR (ATM- and Rad3-Related), ATM (ataxia-telangiectasia mutated), and DNA-PKCs (DNA-dependent protein kinase) are the most upstream kinases of DDR signaling pathway (Blackford and Jackson, 2017Mol Cell. Jun 15;66(6):801-817). An IP-Masspec experiment to find the interacting partners of vGAF was performed. ATR and its obligate interactor ATRIP in vGAF IP mass-spec data was identified. Further, it was found all three RPA70, RPA14 and RPA32 proteins that form complex with ssDNA and recruit ATR/ATRIP complex to the site of DNA damage/replication fork (Zou And Elledge, 2003 Science 6 June;VOL 300). It was found that several other DNA repair proteins that are involved in DNA break repair including Ku60 and Ku80, suggesting a physical association between vGAF and DNA repair machinery (FIG. 1).

### Example 2

### Effect of vGAF on DNA damage repair

To check if vGAF could affect the process of DNA-damage repair, the following experiment was carried out. The phosphorylated form of histone variant H2AX (γ-H2AX) has been used as a marker for DNA damage (Kuo and Yang, 2008In Vivo. May-Jun;22(3):305-9.). The levels of γ-H2AX using western blotting were measured and used as a measure for the level of DNA damage in the tissues or cells used for protein extract preparation. vGAF has been shown to express predominantly in adult mouse skin (Galera, et al., 1996; Journal of Biological Chemistry 271(35):21331-21339 August). Protein extracts prepared after 3-hours of UV treatment from the skin explant shows a higher accumulation of γ-H2AX in vGAF KO (knockout) skin compared to the wild-type controls, suggesting a delayed repair of DNA-damage in KO skin tissue compared to wild-type tissue. It was further checked for the survival of skin primary fibroblast 4-days after the UV treatment using MTT assay. Here again, it was observed that primary fibroblast cells from vGAF KO skin are more sensitive to UV treatment compared to the control. To further confirm the results, the primary fibroblast cells were derived from lung which is another organ where vGAF shows a significant expression. Protein extracts prepared after 3-hours of UV treatment of primary lung fibroblasts showed an increased signal of γ-H2AX in western blots compared to the wild-type lung primary fibroblast cells. These results showed that vGAF is essential for efficient DNA repair after UV induced DNA damage.

Figure 1 demonstrates that vGAF is essential for efficient DNA repair and cell survival after UV induced DNA damage. [A] vGAF KO mice skin explants show higher levels of DNA damage (increased Y-H2Ax)) after UV induced DNA damage. Skin explants were treated with UVC (60J/m2) and allowed to recover for 3 hours. Increased Y-H2Ax was detected in protein extracts prepared from vGAF KO mice skin explants. KO stands for Knock Out, while WT stands for the Wild Type.

[B] Skin primary fibroblast cells derived from vGAF KO mice are sensitive to UVC treatment. Skin primary fibroblast cells were treated with UVC (30J/m2). Cell survival assay shows a significant decrease in survival of primary fibroblast derived from skin of KO mice.

[C] Lung primary fibroblast derived from vGAF KO mice show higher levels of DNA damage (increased Y-H2Ax) after UV induced DNA damage. Lung primary fibroblast were treated with UVC (5J/m2) and were allowed to recover for 3 hours. Increased Y-H2Ax was detected in protein extracts prepared from cells derived from vGAF KO.

### Example 3

### Expression of vGAF is downregulated in skin cancer tissue

UV induced DNA damage is the primary cause of skin cancers, therefore the expression data of vGAF (ZBTB7B) and several other DNA repair proteins (XRCC1, XRCC5, XRCC6, XRCC7, APLF, APRATAXIN, PNKP, Lig4, Lig3, PARP2, and ATR) in skin cutaneous melanoma (SCM) and normal skin tissue (NST) was compared.

GEPIA (Gene Expression Profiling Interactive Analysis) is a web-based tool for the analysis of TCGA (The Cancer Genome Atlas) and GTEx (Genotype-Tissue Expression) database (Tang, et al., 2017). Gene expression data from these two databases using GEPIA was analyzed and it was found that SCM expresses vGAF at a much lower level than the NST, while other DNA repair proteins which were taken in this study do not show a statistically significant change in their expression levels across SCM vs. NST. Further analysis also shows that vGAF expression does not change significantly across all the stages of SCM suggesting a consistent downregulation of vGAF even in the stage 0 of SCM. Collectively, these results suggest that vGAF is heavily downregulated in SCM and low levels of vGAF in skin biopsies can be used as a diagnostic biomarker for SCM.

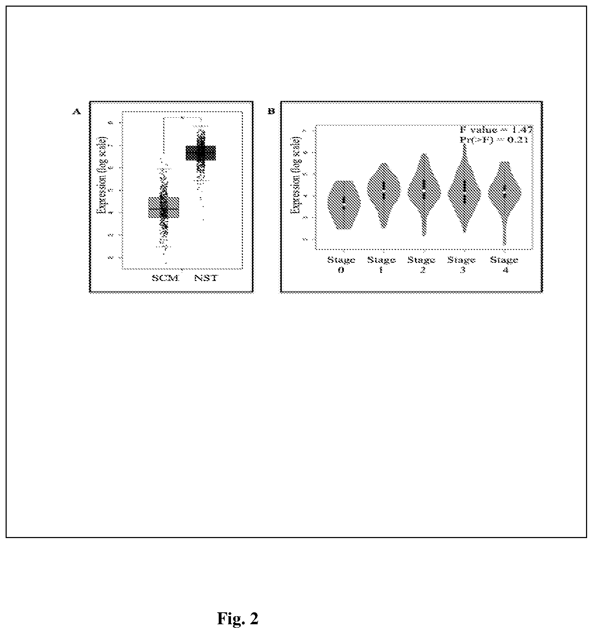

Figure 2. Represents an analysis of vGAF expression in skin cutaneous melanoma (SCM) samples from human subjects
[A] vGAF expression is downregulated in SCM samples compared to normal skin tissue (NST). GEPIA is an interactive web application for gene expression analysis. GEPIA was used to analyze the expression data from 461 SCM and 558 NST samples. Analysis shows a significant decrease in vGAF expression in SCM (p<=0.05).
[B] vGAF expression levels does not change significantly across different stages of SCM. Violin plots of vGAF expression data across pathological stages of SCM show a consistent downregulation including stage 0 of SCM.

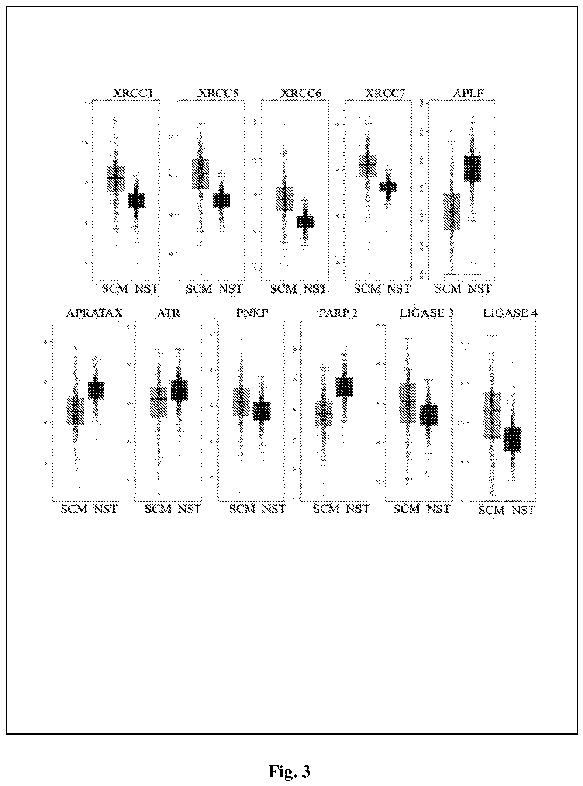

Figure 3. Depicts the GEPIA Analysis for expression data of DNA damage associated proteins in SCM vs. NST

GEPIA was used to analyze the expression data of DNA damage associated proteins (XRCC1, XRCC5, XRCC6, XRCC7, APLF, APRATAXIN, ATR, PNKP, PARP2, LIGASE 3, LIGASE 4) from 461 SCM and 558 NST samples. The expression levels of above-mentioned genes were not found to be statistically different in two groups.

### Example 4

### Overexpression of vGAF in Human Embryonic Kidney (HEK) cells results in efficient DNA repair after UV induced DNA repair

An overexpression study of vGAF in HEK cells was performed to check the effect of vGAF over-expression on efficiency of DNA repair.

Western blotting for gH2AX was used as a proxy for DNA repair. FLAG-tag vGAF or GFP expressing plasmids were used to transfect HEK cells. 36 hours post transfections exponentially growing cultures of HEK cells were exposed to UVC (30J/m2) in UVC cross-linker. 1-hour post UV irradiations cells were re-suspended in cell-lysis buffer. Protein extracts were prepared and resolved on 12% acrylamide gel followed by western blotting. Blots were cut in region of 15kD and probed for gH2AX. Another portion of the blot in the region of 37kD was probed for GAPDH to show equal loading of protein extract.

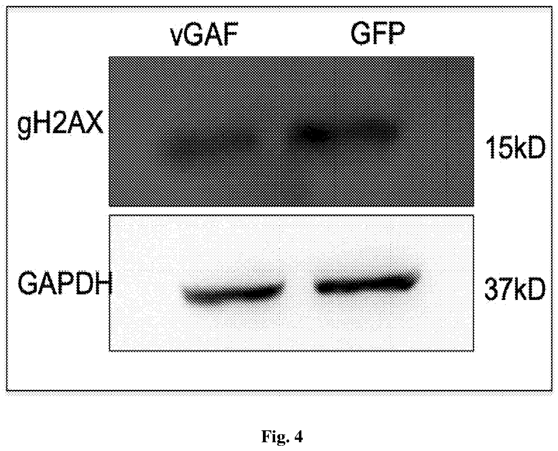

Figure 4 sums up the results of western blots demonstrating efficient DNA repair after UV induced DNA repair with overexpression of vGAF. Protein extracts prepared from vGAF transfected cell and from GFP transfected cells were loaded in lane 1 and lane 2 respectively. Probing of blot with gH2AX antibody shows a decrease in the intensity of gH2AX signals in cell extract prepared with vGAF transfected cells showing low level of damaged DNA. It suggests that vGAF transfected cells could repair DNA damage efficiently compared to control transfected cells.

## Claims

1. An isolated polypeptide, which comprises an amino acid sequence with at least 95% identity to SEQ ID No.: 3, or a polynucleotide encoding said polypeptide for use in a therapeutical method of enhancing the repair of damaged DNA bases in a cell, the method comprising the step of introducing an effective amount of said isolated polypeptide or by introducing said polynucleotide encoding said polypeptide into the cell, wherein the effective amount of polypeptide or polynucleotide is sufficient to enhance the repair of damaged DNA in the cell.

2. Isolated polypeptide for use in the method of claim 1, wherein the cell has been exposed to, or is at risk of being exposed to a DNA damaging agent.

3. An isolated polypeptide, which comprises an amino acid sequence with at least 95% identity to SEQ ID No.: 3, or a polynucleotide encoding said polypeptide for use in a method of preventing melanoma or skin cutaneous melanoma (SCM) occurrence in human skin cells, the method comprising the step of introducing an effective amount of said polypeptide or said polynucleotide into the human skin cells, wherein the effective amount of said polypeptide or said polynucleotide is sufficient to enhance the repair of damaged DNA in the human skin cells, and wherein the human skin cells have been exposed to or are at risk of being exposed to a DNA damaging agent.

4. An isolated polypeptide, which comprises an amino acid sequence with at least 95% identity to SEQ ID No.: 3, or a polynucleotide encoding said polypeptide for use in a method of treating early stage melanoma or early stage skin cutaneous melanoma (SCM) in a patient, the method comprising the step of introducing an effective amount of said polypeptide or said polynucleotide into the early stage melanoma or early stage skin cutaneous melanoma cells, wherein the effective amount of said polypeptide or said polynucleotide is sufficient to enhance the repair of damaged DNA in the early stage melanoma or early stage skin cutaneous melanoma (SCM) cells.

5. A pharmaceutical composition comprising the polypeptide as defined by claim 1 along with the pharmaceutically acceptable carrier for use in a method of prevention or treatment of melanoma.

## Patentansprüche

1. Ein isoliertes Polypeptid, das eine Aminosäuresequenz mit mindestens 95 % Identität mit SEQ ID Nr.: 3 umfasst, oder ein Polynukleotid, das für das Polypeptid kodiert, zur Verwendung in einem therapeutischen Verfahren zur Verbesserung der Reparatur von geschädigten DNA-Basen in einer Zelle, wobei das Verfahren den Schritt des Einführens einer wirksamen Menge des isolierten Polypeptids oder des Einführens des Polynukleotids, das für das Polypeptid kodiert, in die Zelle umfasst, wobei die wirksame Menge des Polypeptids oder Polynukleotids ausreicht, um die Reparatur von geschädigter DNA in der Zelle zu verbessern.

2. Isoliertes Polypeptid zur Verwendung in dem Verfahren nach Anspruch 1, wobei die Zelle einem DNA-schädigenden Agens ausgesetzt war oder Gefahr läuft, dass sie einem solchen ausgesetzt wird.

3. Isoliertes Polypeptid, das eine Aminosäuresequenz mit mindestens 95 % Identität mit SEQ ID No. umfasst: 3, oder ein Polynukleotid, das für dieses Polypeptid kodiert, zur Verwendung in einem Verfahren zur Verhinderung des Auftretens eines Melanoms oder eines kutanen Hautmelanoms (SCM) in menschlichen Hautzellen, wobei das Verfahren den Schritt des Einführens einer wirksamen Menge des Polypeptids oder des Polynukleotids in die menschlichen Hautzellen umfasst, wobei die wirksame Menge des Polypeptids oder des Polynukleotids ausreicht, um die Reparatur von geschädigter DNA in den menschlichen Hautzellen zu verbessern, und wobei die menschlichen Hautzellen einem DNA-schädigenden Agens ausgesetzt waren oder Gefahr laufen, einem solchen ausgesetzt zu werden.

4. Isoliertes Polypeptid, das eine Aminosäuresequenz mit mindestens 95 % Identität mit SEQ ID No. umfasst: 3, oder ein Polynukleotid, das für das Polypeptid kodiert, zur Verwendung in einem Verfahren zur Behandlung eines Melanoms im Frühstadium oder eines kutanen Hautmelanoms im Frühstadium (SCM) bei einem Patienten, wobei das Verfahren den Schritt des Einführens einer wirksamen Menge des Polypeptids oder des Polynukleotids in die Melanomzellen im Frühstadium oder die kutanen Hautmelanomzellen im Frühstadium umfasst, wobei die wirksame Menge des Polypeptids oder des Polynukleotids ausreicht, um die Reparatur geschädigter DNA in den Zellen des Melanoms im Frühstadium oder des kutanen Hautmelanoms (SCM) im Frühstadium zu verbessern.

5. Eine pharmazeutische Zusammensetzung, die das Polypeptid nach Anspruch 1 zusammen mit einem pharmazeutisch annehmbaren Träger zur Verwendung in einem Verfahren zur Vorbeugung oder Behandlung von Melanomen umfasst.

## Revendications

1. Polypeptide isolé, qui comprend une séquence d'acides aminés présentant au moins 95 % d'identité avec SEQ ID No. : 3, ou polynucléotide codant pour ledit polypeptide pour une utilisation dans une méthode thérapeutique d'amélioration de la réparation de bases d'ADN endommagées dans une cellule, la méthode comprenant l'étape d'introduction d'une quantité efficace dudit polypeptide isolé ou par introduction dudit polynucléotide codant pour ledit polypeptide dans la cellule, dans lequel la quantité efficace de polypeptide ou polynucléotide est suffisante pour améliorer la réparation de l'ADN endommagé dans la cellule.

2. Polypeptide isolé pour une utilisation dans la méthode selon la revendication 1, dans lequel la cellule a été exposée, ou est à risque d'être exposée à un agent endommageant l'ADN.

3. Polypeptide isolé, qui comprend une séquence d'acides aminés ayant au moins 95 % d'identité avec SEQ ID No. : 3, ou polynucléotide codant pour ledit polypeptide pour une utilisation dans une méthode de prévention de l'apparition d'un mélanome ou d'un mélanome cutané (SCM) dans des cellules de la peau humaine, le procédé comprenant l'étape d'introduction d'une quantité efficace dudit polypeptide ou dudit polynucléotide dans les cellules de peau humaine, dans lequel la quantité efficace dudit polypeptide ou dudit polynucléotide est suffisante pour améliorer la réparation de l'ADN endommagé dans les cellules de peau humaine, et dans lequel les cellules de peau humaine ont été exposées ou sont à risque d'être exposées à un agent endommageant l'ADN.

4. Polypeptide isolé, qui comprend une séquence d'acides aminés ayant au moins 95 % d'identité avec SEQ ID No. : 3, ou polynucléotide codant pour ledit polypeptide pour une utilisation dans une méthode de traitement d'un mélanome à un stade précoce ou d'un mélanome cutané à un stade précoce (SCM) chez un patient, la méthode comprenant l'étape d'introduction d'une quantité efficace dudit polypeptide ou dudit polynucléotide dans les cellules de mélanome à un stade précoce ou de mélanome cutané à un stade précoce, dans lequel la quantité efficace dudit polypeptide ou dudit polynucléotide est suffisante pour améliorer la réparation de l'ADN endommagé dans les cellules de mélanome à un stade précoce ou de mélanome cutané (SCM) à un stade précoce.

5. Composition pharmaceutique comprenant le polypeptide tel que défini par la revendication 1 ainsi que le support pharmaceutiquement acceptable pour une utilisation dans une méthode de prévention ou de traitement du mélanome.
